# EUROPEAN PATENT SPECIFICATION

(11) **EP 3 302 383 B1**
(45) Date of publication and mention of the grant of the patent: **11.09.2019**
(21) Application number: 16726783.0
(22) Date of filing: 27.05.2016
(51) Int. Cl.: A61F 13/06

(54) **A CORN DRESSING**
HÜHNERAUGENPFLASTER
PANSEMENT COR

(30) Priority: 29.05.2015 DK 201570329
(43) Date of publication of application: 11.04.2018
(73) Proprietor: Coloplast A/S, 3050 Humlebæk (DK)
(72) Inventor: IGWEBUIKE, Henning, 3540 Lynge (DK); HANSEN, Grazyna, 2000 Frederiksberg C (DK)
(86) International application number: PCT/DK2016/050155
(87) International publication number: WO 2016/192732

(56) References cited:
- DE-C- 889 678
- GB-A- 515 244
- US-A1- 2006 105 028
- US-A1- 2010 312 159
- US-B1- 6 270 872
- US-B1- 6 471 986

## Description

### Summary of the Invention

One aspect provides a corn dressing for application to a corn. The dressing comprises an adhesive layer having a skin-facing surface and a non-skin-facing surface. The non-skin-facing surface is provided with a backing layer. The dressing comprises a central portion and a border portion surrounding the central portion and a central hole or depression. The central portion comprises a core surrounding the central hole or depression. The core is pressure resistant and is embedded in the adhesive layer.

### Brief Description of the Drawing

The accompanying drawings are included to provide a further understanding of embodiments.
Figure 1 illustrates an embodiment of a corn dressing seen from above,
Figure 2 shows the embodiment of Figure 1 in cross-section,
Figure 3 shows an embodiment of a core of a corn dressing seen from above,
Figure 4 shows the embodiment of Figure 3 in cross-section,
Figure 5 shows a 15 minutes pressure test of Sample A and Sample B,
Figure 6 shows a 24 hours pressure test of Sample B and
Figure 7 shows a 24 hours pressure test of Sample A.

### Detailed Description of the Invention

In the following detailed description, reference is made to the accompanying drawings. The drawings form a part of this specification and illustrate exemplary embodiment for practicing the invention.

One aspect is to provide a corn dressing comprising an adhesive layer having a skin-facing surface and a non-skin-facing surface, the non-skin-facing surface being provided with a backing layer, the dressing comprises a central portion and a border portion surrounding the central portion, the central portion comprises a central hole or depression, the central portion comprises a core surrounding the central hole or depression, the core is pressure resistant and wherein the core is at least partly embedded in the adhesive layer.

By "pressure resistant" is herein meant that the material is not plastic and will not flow when exposed to pressure. However, the core may be elastic but still able to maintain its shape under pressure. The core material may have a tan(δ) of less than 0.8 at 25°C and 1 Hz in order to avoid cold flowing. The material may be elastic in order to be capable of conforming to the contours of the body, such as being fitted around a toe.

The core material may have a Shore M hardness of less than 60, such as 50-20, such as 40-20, such as 38-22 or even 24-36.

Corns may appear where the skin is exposed to pressure. Thus, a corn dressing applied to the corn may also be exposed to this pressure. A corn dressing having a central hole or depression for accommodating the corn may relieve this pressure from the corn and provide a cushioning effect to the area around the corn.

The core may be made from one or more elastomers or elastic material. Examples of suitable elastomers may be styrenic block copolymers, polyolefin blends, thermoplastic polyurethanes, thermoplastic copolyester, thermoplastic polyamides or blends thereof.

In embodiments, the core is non-adhesive.

In embodiments, the core may comprise an adhesive or it may show adhesive properties.

The core may be non-absorbent.

In embodiments, the backing layer is moldable. By moldable is herein meant that the backing layer may be molded into a desired shape by treatment with heat.

The core comprises an inner boundary neighboring the central hole or depression and an outer boundary defined as the radially outwardly extending edge. The inner boundary of the core may be substantially circular, thereby matching the shape of a corn.

In embodiments, the outer boundary is substantially circular. The core may be ring-shaped.

In embodiments, the outer boundary of the core is substantially rhombus shaped.

The core may be beveled along the inner and/or outer boundary. The beveling smoothens sharp edges and may thereby decrease the risk of pressure marks.

In embodiments, the core comprises a ring-shaped portion. The core may further comprise at least one radially extending flange, extending away from the core. The flange may be beveled to a thickness smaller than the thickness of the ring-shaped part of the core.

In embodiments, the thickness of the adhesive layer in the section overlying the ring-shaped portion of the core is less than 0.2 mm.

In embodiments, the thickness of the ring-shaped portion of the core at the thickest point is at least 0.8 mm, such as at least 1 mm, 1.2 mm, 1.5 mm, 1.7 mm or even at least 2 mm.

The thickness is understood as the distance measured in a direction being perpendicular to the skin-facing surface.

In embodiments, the diameter of the outer periphery of the ring-shaped portion of the core may be 5-8 mm such as 6-7 mm. The diameter of the inner periphery may be 3-5 mm, such as 4 mm. The diameter of the inner and outer periphery is measured in the plane of the skin facing surface.

The hydrocolloid layer next to and surrounding the outer periphery of the core may have approximately the same thickness as the core. The hydrocolloid layer may be beveled at the outer edge portion in order to provide a smooth transition to the skin and avoid pressure marks.

In embodiments, the thickness of the adhesive layer is thicker at the peripheral portion than at the central portion. The adhesive layer may be quite thin at the central portion overlying the core and the central depression, thereby hindering adhesive flowing into the central depression. The thicker layer at the border portion encircling the core provides broader distribution of pressure and an improved attachment to the skin. Furthermore, embedding the core in the adhesive provides improved pressure distribution and the core is less prone to detaching from the backing layer.

In embodiments, a surface area of the core may constitute a part of the skin-contacting surface such that the adhesive layer and the surface of the core are in same level at the skin-contacting surface of the dressing. The core may be embedded in the adhesive layer in such a way that the skin facing surface of the core and the skin facing surface of the adhesive are in line, providing a continuous, flat skin-contacting surface for attachment to the skin.

The core may be embedded in the adhesive layer such that only the skin-facing surface of the elastic core is not covered by the adhesive layer. Thus, the non-skin-facing surface of the core and the side portions connecting the skin-facing and the non-skin facing surface may be covered by adhesive such that the adhesive is surrounding the core, optionally with the exception of the skin-facing surface of the core. In embodiments, the skin-facing surface of the elastic core may be covered with a thin layer of adhesive.

In embodiments, the core may be partially embedded in the adhesive layer.

The edge portion of the dressing may be beveled. The beveling may provide a smooth transition to the skin and minimize adhesive edge portions. The beveling further facilitates a gradual decrease of pressure, thereby reducing the risk of pressure marks.

In embodiments, the central hole or depression may comprise an active agent for degrading the corn.

In embodiments, at least the central hole or depression of the dressing may be transparent or translucent, thereby enabling easy and precise application of the dressing to the corn.

The adhesive may be any suitable skin-friendly adhesive. The adhesive may be non-absorbent or it may comprise absorbent particles.

The adhesive may be any skin-friendly adhesive known per se for production of medical articles, which are to be adhered to human skin, preferably an adhesive comprising hydrocolloids or other moisture absorbing constituents for prolonging the time of use. The adhesive may suitably be of the type disclosed in US patent Nos. 4,231,369, 4,367,732, 4,867,748, and 5,714,225. Especially preferred are the adhesives disclosed in US patent Nos. 4,367,732, and 5,714,225.

In embodiments, the adhesive is an absorbent adhesive, such as a hydrocolloid adhesive. By absorbent adhesive is meant that the adhesive layer may comprise hydrocolloid particles or super absorbent particles or fibers. The presence of hydrocolloid in the adhesive provides a good environment for moist wound healing as well as for other skin conditions as well as for handling moisture from the skin, such as perspiration. By incorporating an amount of hydrocolloid in the adhesive, the corn dressing is able to handle moisture in most conditions.

In embodiments, suitable hydrocolloids for the dressing may include synthetic polymers prepared from single or multiple monomers, naturally occurring hydrophilic polymers or chemically modified naturally occurring hydrophilic polymers. The hydrocolloid polymers may be linear or cross-linked. This include natural or chemically modified natural polymers like cellulosics such as CMC, chitosan, pectin, guar gum, starches or dextrines, collagenes and gelatine and synthetic polymers like polyacrylic acid, polyvinylealcohol/acetate, polyhydroxy-alkyl acrylates and methacrylates, polyacrylamides, polystyrene sulfonates, polyvinyl pyrilidone, polyglycols, copolymers, grafts of such, copolymers or compositions of such.

### Detailed Description of the Drawing

In the following detailed description, reference is made to the accompanying drawings. The drawings form a part of this specification and illustrate exemplary embodiments for practicing the invention. Directional terminology, such as "top," "bottom," "front," "back," "leading," "trailing," etc., is used with reference to the orientation of the Figure(s) being described. Because components of embodiments can be positioned in a number of orientations, the directional terminology is used for purposes of illustration and is in no way limiting. It is to be understood that other embodiments may be utilized and structural or logical changes may be made without departing from the scope of the invention. The detailed description describes examples for practicing the invention and is not to be read to limit the scope of the invention. The scope of the invention is defined by the attached claims.

Embodiments, and features of the various exemplary embodiments described in this application, may be combined with each other ("mixed and matched"), unless specifically noted otherwise.

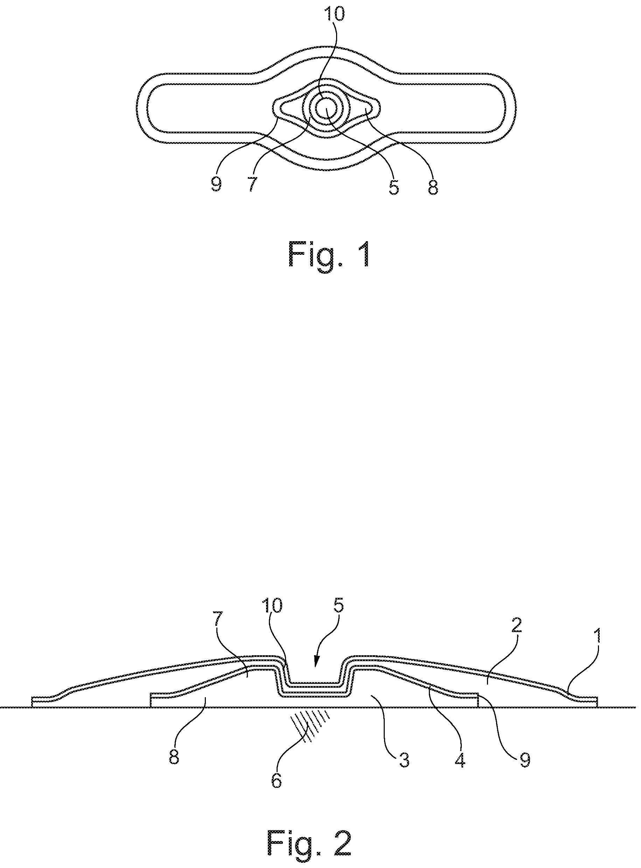

In Figure 1 and Figure 2 is shown an embodiment of a corn dressing, comprising a backing layer 1 and an adhesive layer 2. The backing layer 1 is coated with the adhesive 2 on the skin-facing surface. The adhesive 2 may be a hydrocolloid adhesive. At the central portion of the dressing is a pressure resistant core 3 embedded in the adhesive layer 2 such that the skin-facing surface of the core 3 is flush with the skin-facing surface of the adhesive layer, providing a continuous and planar skin-contacting surface. The entire surface of the core except the skin-facing surface is covered with the adhesive. The core 3 may optionally be provided with a cover layer 4 on the non-skin facing surface. The central portion of the dressing comprises a central depression 5. Alternatively, the central portion may be provided with a central hole penetrating all layers of the dressing or penetrating the core 3 and optional cover layer 4. The hole or depression 5 ensures that the underlying corn 6 is relieved from pressure. The edges of the inner boundary 10 of the core 3 may be beveled. The core 3 comprises a ring-shaped structure 7 provided with axially extending beveled flanges 8. The flanges 8 may extend further in longitudinal direction of the dressing. The outer boundary 9 of the core 3 may have a substantially rhombus shape. The inner boundary 10 of the core 3 may be substantially circular, encircling the corn 6. The adhesive layer 2 extends over the non-skin facing surface of the core 3. The thickness of the adhesive layer 2 at the central depression 5 and over the ring-shaped structure 7 of the core 3 is minimized in order to avoid the adhesive 2 to flow into the depression 5, but enough to attach the backing layer 1 to the core 3. The adhesive layer of the peripheral portion of the dressing is thicker, thereby providing a cushioning effect as well as it may smoothen the outer boundary of the core 3. The outer boundary of the dressing is beveled in order to minimize adhesive edges.

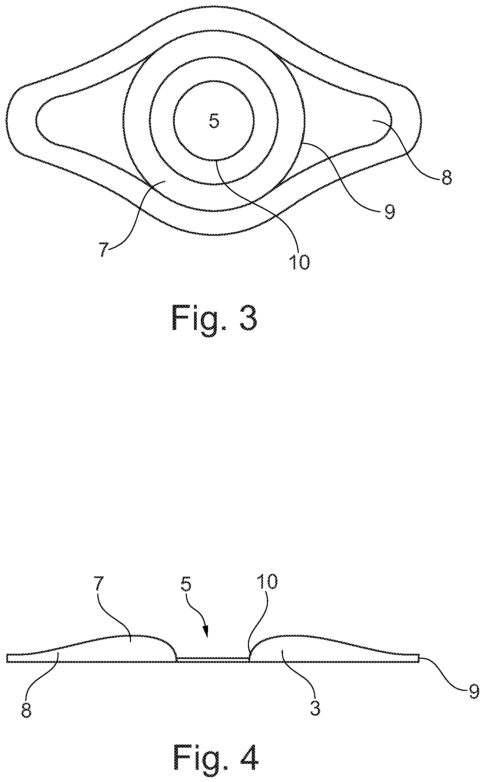

Figure 3 and 4 show the core 3, without a cover layer 4 and before embedding the core 3 into the adhesive 2 of the dressing. The core 3 may be embedded in a dressing of any suitable shape, dependent of which part of the body it is to be applied to. An elongated shape as shown in Figure 1 and 2 may be suitable for application to toes whereas a more round shape may suit the plantar of the foot.

### Examples

### EXAMPLE 1

A test was performed in order to observe the behavior of the samples when exposed to a constant pressure for 15 minutes.

Sample A: Corn dressing comprising a polyurethane backing layer coated with a hydrocolloid adhesive and a core of a pressure resistant elastomer embedded in the hydrocolloid adhesive. The core is made from 92,5 % Kraton D1161 and 7,5 % DOA (dioctyl adipate). The core has a thickness of 0.8 mm, an inner width of 4 mm and an outer width of 7 mm. The adhesive layer over the core and at the central portion of the dressing is 0.2 mm thick and 1.0 mm thick at the peripheral portion.

Sample B: Corn dressing comprising a polyurethane backing layer coated with a hydrocolloid adhesive, having the same outline and dimensions as Sample A but where the pressure resistant elastomer core was absent and substituted with hydrocolloid adhesive instead.

The measurements were performed by placing a sample on an I-scan 2-dimensional pressure detector and a weight of 2176 g was placed on top of the sample. Thereby, the pressure on the opposite side of the dressing was measured and followed over time.

The objective of the test was to see whether the dressing could maintain its shape while under pressure and thereby offer protection against pressure. If the pressure distribution showed a ring-shaped mark where there is no pressure in the middle, the protection is optimal, as the pressure on the corn then was minimized.

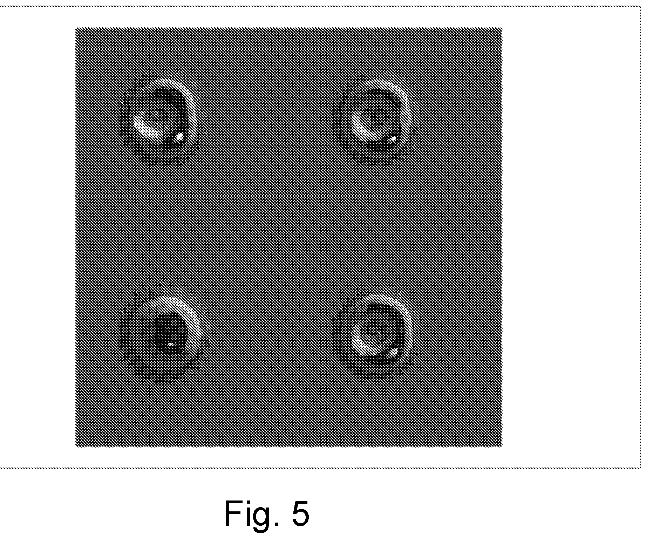

In Figure 5 is shown the result of the pressure distribution in the samples, measured at start (top row) and after 15 minutes under pressure (bottom row). Sample A is to the right and Sample B is to the left. As can be seen from the Figures, both samples showed a ring-shaped pressure mark at the beginning of the test. However, after 15 minutes under load, the picture was different. Where the pressure mark of Sample A still was ring-shaped and almost equal to the picture at the beginning of the test, the pressure mark of Sample B (control sample) had changed substantially, the pressure now being highest at the center of the dressing. The hydrocolloid adhesive of Sample B was not able to maintain its shape and the product was no longer able to provide protection and pressure relief to a corn as the hydrocolloid adhesive had flown into the central depression. On the contrary, the pressure was highest at the center of the sample, being right over the corn. Sample A maintained its shape during the test and provided pressure relief over the corn.

### EXAMPLE 2

A test was performed in order to observe the behavior of the samples when exposed to a constant pressure for 24 hours.

Samples were equal to Sample A and B of Example 1.

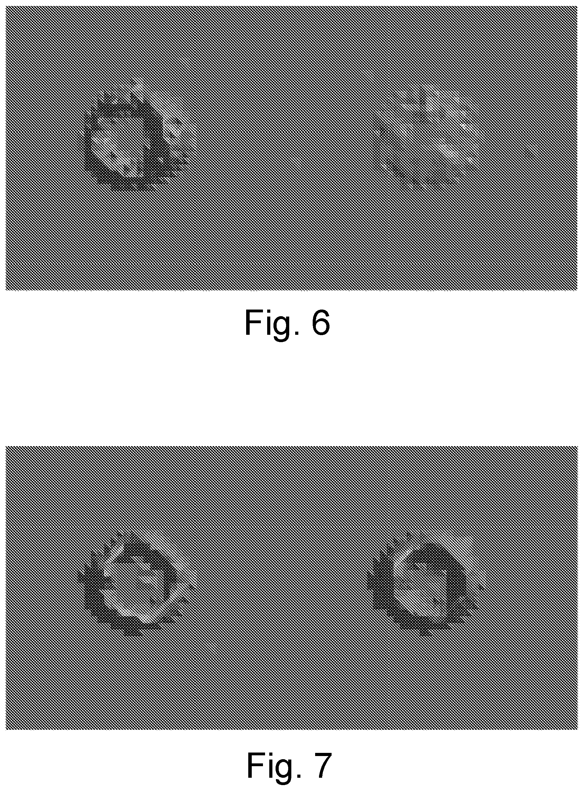

The measurements were performed by placing the sample on an I-scan 2-dimensional pressure detector and a weight of 1089 g was placed on top of the sample. Thereby, the pressure on the opposite side of the bandage was measured and followed over time. The results are shown in Figure 6 (Sample B) and Figure 7 (Sample A).

As can be seen from Figure 7, the elastomer core in Sample A maintained its shape and provided pressure relief over the corn even after 24 hours of constant pressure, shown as a ring-shaped pressure mark in the test.

Figure 6 shows that the hydrocolloid adhesive of the Sample B had flown into the center of the dressing when under pressure, thereby causing the pressure to rise at the central portion of the dressing, and the dressing thus failed to relieve pressure over the corn.

### METHODS

### Determination of tan(δ).

The parameter tan(δ) defined as tan(δ)=G"/G', was used as a measure of the hardness/softness of the core material. Tan(δ) was measured as follows: A plate of a core material was pressed into a layer of 1 mm thickness. A round sample of 25 mm in diameter was cut out and placed in a RheoStress RS600 rheometer from Thermo Electron. The geometry applied was parallel plates 25 mm and the deformation was fixed at 1% to ensure that measurements were in the linear regime. The measurement was carried out at 1 Hz and 25°C.

## Claims

1. A corn dressing comprising an adhesive layer having a skin-facing surface and a non-skin-facing surface, the non-skin-facing surface being provided with a backing layer, the dressing comprises a central portion and a border portion surrounding the central portion, the central portion comprises a central hole or depression and a core surrounding the central hole or depression, wherein the core is pressure resistant being able to maintain its shape under pressure and wherein the core is embedded in the adhesive layer and wherein the thickness of the adhesive layer is thicker at the peripheral portion than at the central portion.

2. Corn dressing according to claim 1, wherein the core is made from one or more elastomers.

3. Corn dressing according to claim 2, wherein the elastomer is chosen from the group of styrenic block copolymers, polyolefin blends, thermoplastic polyurethanes, thermoplastic copolyester, thermoplastic polyamides and blends thereof.

4. Corn dressing according to the preceding claims, wherein the core comprises an adhesive.

5. Corn dressing according to the preceding claims, wherein the core is non-absorbent.

6. Corn dressing according to any of the preceding claims, wherein a surface area of the core constitutes a part of a skin-contacting surface of the dressing.

7. Corn dressing according to any of the preceding claims, wherein the border portion is beveled.

8. Corn dressing according to the preceding claims, wherein the core comprises a ring-shaped portion.

9. Corn dressing according to claim 8, wherein the thickness of the adhesive layer over the ring-shaped portion of the core is less than 0.2 mm.

10. Corn dressing according to claim 8 or 9, wherein the thickness of the ring-shaped portion of the core is at least 0.8 mm.

11. Corn dressing according to the preceding claims, wherein the central hole or depression comprises an active agent for degrading the corn.

12. Corn dressing according to the preceding claims, wherein the adhesive is a hydrocolloid adhesive.

13. Corn dressing according to the preceding claims, wherein an outer boundary of the core is circular.

14. Corn dressing according to any of claims 1-12, wherein an outer boundary of the core is rhombus shaped.

15. Corn dressing according to the preceding claims, wherein an inner boundary of the core is circular.

## Patentansprüche

1. Hühneraugenpflaster, umfassend eine Klebeschicht mit einer der Haut zugewandten Oberfläche und einer nicht der Haut zugewandten Oberfläche, wobei die nicht der Haut zugewandte Oberfläche mit einer Trägerschicht versehen ist, wobei das Pflaster einen zentralen Teil und einen den zentralen Teil umgebenden Randteil umfasst, wobei der zentrale Teil ein zentrales Loch oder eine zentrale Vertiefung und einen das zentrale Loch bzw. die zentrale Vertiefung umgebenden Kern umfasst, wobei der Kern dahingehend druckresistent ist, als dass er unter Druck seine Form beibehält, und wobei der Kern in der Klebeschicht eingebettet ist und wobei die Dicke der Klebeschicht am peripheren Teil dicker ist als am zentralen Teil.

2. Hühneraugenpflaster nach Anspruch 1, wobei der Kern aus einem oder mehreren Elastomeren hergestellt ist.

3. Hühneraugenpflaster nach Anspruch 2, wobei das Elastomer aus der aus Styrol-Blockcopolymeren, Polyolefinmischungen, thermoplastischen Polyurethanen, thermoplastischem Copolyester, thermoplastischen Polyamiden und Mischungen davon bestehenden Gruppe ausgewählt ist.

4. Hühneraugenpflaster nach den vorhergehenden Ansprüchen, wobei der Kern einen Klebstoff umfasst.

5. Hühneraugenpflaster nach den vorhergehenden Ansprüchen, wobei der Kern nicht absorbierend ist.

6. Hühneraugenpflaster nach einem der vorhergehenden Ansprüche, wobei die Oberfläche des Kerns Teil einer mit der Haut in Kontakt stehenden Oberfläche des Pflasters ist.

7. Hühneraugenpflaster nach einem der vorhergehenden Ansprüche, wobei der Randteil abgeschrägt ist.

8. Hühneraugenpflaster nach den vorhergehenden Ansprüchen, wobei der Kern einen ringförmigen Teil umfasst.

9. Hühneraugenpflaster nach Anspruch 8, wobei die Dicke der Klebeschicht über dem ringförmigen Teil des Kerns weniger als 0,2 mm beträgt.

10. Hühneraugenpflaster nach Anspruch 8 oder 9, wobei die Dicke des ringförmigen Teils des Kerns mindestens 0,8 mm beträgt.

11. Hühneraugenpflaster nach den vorhergehenden Ansprüchen, wobei das zentrale Loch bzw. die zentrale Vertiefung einen Wirkstoff zum Abbau des Hühnerauges umfasst.

12. Hühneraugenpflaster nach den vorhergehenden Ansprüchen, wobei es sich bei dem Klebstoff um einen Hydrokolloidklebstoff handelt.

13. Hühneraugenpflaster nach den vorhergehenden Ansprüchen, wobei ein äußerer Rand des Kerns kreisförmig ist.

14. Hühneraugenpflaster nach einem der Ansprüche 1-12, wobei ein äußerer Rand des Kerns rhombusförmig ist.

15. Hühneraugenpflaster nach einem der vorhergehenden Ansprüche, wobei ein innerer Rand des Kerns kreisförmig ist.

## Revendications

1. Pansement pour cors comprenant une couche d'adhésif ayant une surface orientée vers la peau et une surface non orientée vers la peau, la surface non orientée vers la peau étant pourvue d'une couche de support, le pansement comprenant une partie centrale et une partie marginale entourant la partie centrale, la partie centrale comprenant un trou ou un creux central et un élément central entourant le trou ou le creux central, l'élément central étant résistant à la pression et capable de conserver sa forme sous pression et l'élément central étant incorporé dans la couche d'adhésif, et la couche d'adhésif étant plus épaisse dans la partie périphérique que dans la partie centrale.

2. Pansement pour cors selon la revendication 1, dans lequel l'élément central se compose d'un ou plusieurs élastomères.

3. Pansement pour cors selon la revendication 2, dans lequel l'élastomère est sélectionné dans le groupe constitué de copolymères à blocs de styrène, de mélanges de polyoléfines, de polyuréthanes thermoplastiques, de copolyesters thermoplastiques, de polyamides thermoplastiques, et de mélanges de ceux-ci.

4. Pansement pour cors selon les revendications précédentes, dans lequel l'élément central comprend un adhésif.

5. Pansement pour cors selon les revendications précédentes, dans lequel l'élément central est non absorbant.

6. Pansement pour cors selon l'une quelconque des revendications précédentes, dans lequel une zone superficielle de l'élément central fait partie d'une surface de contact cutané du pansement.

7. Pansement pour cors selon l'une quelconque des revendications précédentes, dans lequel la partie marginale est biseautée.

8. Pansement pour cors selon les revendications précédentes, dans lequel l'élément central comprend une partie en forme d'anneau.

9. Pansement pour cors selon la revendication 8, dans lequel l'épaisseur de la couche d'adhésif sur la partie en forme d'anneau de l'élément central est inférieure à 0,2 mm.

10. Pansement pour cors selon la revendication 8 ou 9, dans lequel l'épaisseur de la partie en forme d'anneau de l'élément central est d'au moins 0,8 mm.

11. Pansement pour cors selon les revendications précédentes, dans lequel le trou ou le creux central comprend un agent actif pour la dégradation du cor.

12. Pansement pour cors selon les revendications précédentes, dans lequel l'adhésif est un adhésif hydrocolloïde.

13. Pansement pour cors selon les revendications précédentes, dans lequel une limite extérieure de l'élément central est circulaire.

14. Pansement pour cors selon l'une quelconque des revendications 1 à 12, dans lequel une limite extérieure de l'élément central est en forme de losange.

15. Pansement pour cors selon les revendications précédentes, dans lequel une limite intérieure de l'élément central est circulaire.
